# EUROPÄISCHE PATENTSCHRIFT

(11) **EP 0 321 722 B1**
(45) Veröffentlichungstag und Bekanntmachung des Hinweises auf die Patenterteilung: **16.02.1994**
(21) Anmeldenummer: 88119553.1
(22) Anmeldetag: 24.11.1988
(51) Int. Cl.: C07K 1/12, C12P 21/02

(54) **Verfahren zur Herstellung von Dipeptiden**
Process for obtaining dipeptides
Procédé pour l'obtention de dipeptides

(30) Priorität: 08.12.1987 DE 3741515
(43) Veröffentlichungstag der Anmeldung: 28.06.1989
(73) Patentinhaber: Degussa Aktiengesellschaft, 60311 Frankfurt (DE)
(72) Erfinder: Drauz, Karlheinz, Dipl.Chem.Dr.., D-6463 Freigericht 1 (DE); Fürst, Peter, Dr., c/o Universität Hohenheim, D-.... Hohenheim (DE); Groeger, Ulrich, Dipl.Biol.Dr., D-4800 Bielefeld (DE); Leuchtenberger, Wolfgang, Dipl.Chem.Dr., D-4800 Bielefeld 1 (DE); Stehle, Peter, Dr., c/o Universität Hohenheim, D-.... Hohenheim (DE)

(56) Entgegenhaltungen:
- EP-A- 0 017 485
- INTERNATIONAL JOURNAL OF PEPTIDE & PROTEIN RESEARCH, Band 23, 1984; Yu.V. MITIN et al., Seiten 528-534#
- ENZYME NOMENCLATURE, 1984, IVB Academic Press Inc.; pp. 330-355#

## Beschreibung

Die Erfindung betrifft ein Verfahren zur enzymatischen Herstellung von Dipeptiden durch Umsetzung von N-geschützten α-Aminocarbonsäurealkylestern mit einer Aminokomponente in Gegenwart einer Cystein-Protease [EC 3.4.22] bei einem pH von mindestens 7 und bei einer Temperatur im Bereich zwischen Raumtemperatur und 60°C.

Aus der EP-A 0 017 485 ist bekannt, daß Dipeptide durch Umsetzung von N-geschützten α-Aminosäurealkylestern mit freien α-Aminosäuren hergestellt werden können. Die Synthese wird durch Exopeptidasen [EC 3.4.16 + 18] katalysiert.

Peptidsynthesen mit Proteasen sind aus Int. J. Peptide Protein Res. 23, 1984, 528 - 534 bekannt, Dipeptide werden hierbei durch Umsetzung von N-geschützten α-Aminocarbonsäurealkylestern als Carboxylkomponente mit einer Aminokomponente in Gegenwart der Cystein-Protease Papain bei einem pH von mindestens 7 und bei einer Temperatur im Bereich zwischen Raumtemperatur und 50°C erhalten. Als Aminokomponente dienen Amide und t-Butylester von Aminocarbonsäuren. Es wird ausdrücklich festgestellt, daß Aminocarbonsäuren als Aminokomponente ungeeignet sind. Es wird ferner empfohlen, einen Überschuß an der Carboxylkomponente einzusetzen, weil dies notwendig ist, um die Ausbeute an Peptiden zu erhöhen.

Der Nachteil dieses bekannten Verfahrens besteht darin, daß die entstehenden Dipeptide am Carboxylende derivatisiert sind. Die selektive Abspaltung der Schutzgruppe, insbesondere der Amidogruppe, ist ohne gleichzeitige Spaltung der Peptidbindung kaum möglich, so daß man auf diesem Wege nicht zu Dipeptiden mit freier Carboxylgruppe gelangen kann.

Aufgabe der vorliegenden Erfindung ist ein Verfahren zur enzymatischen Herstellung von Dipeptiden mittels einer Protease [EC 3.4.22], das mit geringerem Schutzgruppenaufwand als bei herkömmlichen Protease-unterstützten Peptidsynthesen auskommen soll.

Das erfindungsgemäße Verfahren ist nun dadurch gekennzeichnet, daß man als Aminokomponente eine α-Aminocarbonsäure mit underivatisierter α-Amino- und Carboxylgruppe einsetzt.

Das erfindungsgemäße Verfahren ist besonders geeignet zur Knüpfung einer Peptidbindung zwischen einem N-geschützten α-Aminocarbonsäurealkylester als Carboxylkomponente und einer basischen oder neutralen aliphatischen α-Aminocarbonsäure als Aminokomponente.

Besonders vorteilhaft ist es, wenn man die Aminokomponente in einem molaren Überschuß, bezogen auf die eingesetzte Carboxylkomponente, einsetzt. Der Überschuß kann im allgemeinen das 1- bis 150-fache betragen.

Der entscheidende Vorteil des erfindungsgemäßen Verfahrens liegt darin, daß unmittelbar N-geschützte Dipeptide mit underivatisierter Carboxylendgruppe entstehen. Wird die N-Schutzgruppe in an sich bekannter Weise selektiv abgespalten, erhält man daher problemlos die freien Dipeptide.

Als Carboxylkomponente dienen N-geschützte α-Aminocarbonsäurealkylester, vorzugsweise mit 1 bis 4 Kohlenstoffatomen in der Alkylgruppe, insbesondere die Methyl- und Ethylester. Geeignete N-Schutzgruppen sind vor allem die Benzylgruppe, die Carbobenzoxygruppe,die t-Butyloxycarbonylgruppe (BOC-Gruppe), oder auch die Acetyl- oder Benzoylgruppe.

Als Cystein-Protease können neben dem bevorzugten Papain [ EC 3.4.22.2 ] beispielsweise auch Ficin [ EC 3.4.22.3 ] oder Bromelain [ EC 3.4.22.4 ] verwendet werden. Die Enyzme können dem Reaktionsgemisch in fester, z. B. lyophilisierter, oder in gelöster Form zugesetzt werden. Dabei ist es nicht notwendig, daß die Enzyme in hochgereinigter Form vorliegen. So führt auch der Einsatz von lyophilisierten Rohpräparaten aus Carica papaya, Ficus carica oder Ananas comosus zu befriedigenden Syntheseergebnissen. Es ist jedoch vorteilhaft, wenn die Cystein-Proteasen vor ihrer Verwendung in Gegenwart von 1 bis 10 mM Ethylendiamintetraacetat (EDTA) und wahlweise von 5 bis 20 mM Cystein, Dithiothreitol (DTT) oder ß-Mercaptoethanol 30 bis 60 Minuten lang bei einer Temperatur im Bereich von 25 bis 40 °C aktiviert werden. Diese Behandlung bringt den Vorteil, daß die spezifische Aktivität der Enzyme erhöht und damit die erforderliche Reaktionszeit verkürzt bzw. bei gleicher Reaktionszeit weniger Enzym benötigt wird. Die Enzyme können im Reaktionsgemisch sowohl in freier (nativer) Form als auch an ein Trägermaterial gebunden (immobilisiert) vorliegen.

Die Konzentration der Carboxylkomponente zu Beginn der Reaktion ist nicht kritisch. Die Wahl der Anfangskonzentration wird vielmehr im wesentlichen durch die Löslichkeit der Carboxylkomponente bestimmt. Z.B. wird in dem Konzentrationsbereich zwischen 0,01 und 0,20 M Cbz-Ala-OMe die Ausbeute an dem zu synthetisierenden Dipeptid nicht durch die Anfangskonzentration an Cbz-Ala-OMe beeinflußt. Andererseits können dem Reaktionsgemisch zur Verbesserung der Löslichkeit der teilweise in reinem Wasser nur schlecht löslichen N-geschützten α-Aminocarbonsäurealkylester auch organische Lösungsmittel, wie Methanol, Ethanol, Isopropylalkohol, 1,4-Dioxan, Tetrahydrofuran, Dimethylformamid, Dimethylacetamid, Dimethylsulfoxid, Dimethoxyethan, Tetramethylharnstoff, Dimethylpropylenharnstoff oder N-Methylpyrrolidon zugesetzt werden.

Die Art des organischen Lösungsmittels und seine Menge sollten so gewählt werden, daß die Aktivität und die Stabilität des eingesetzten Enzyms nicht wesentlich beeinträchtigt werden, weil sonst die Ausbeute an dem gewünschten Dipeptid erniedrigt werden könnte.

Das erfindungsgemäße Verfahren wird bei einem pH-Wert im Bereich von 7,0 bis 10,0, vorzugsweise von 8,8 bis 9,5, durchgeführt. Die Reaktionstemperatur kann im Bereich von Raumtemperatur bis 60 °C, vorzugsweise von 35 bis 40 °C, liegen.

Als Aminokomponente werden α-Aminocarbonsäuren mit freier α-Aminogruppe und freier Carboxylgruppe eingesetzt. Zur Vermeidung von unerwünschten Nebenreaktionen kann es zweckmäßig sein, zusätzliche funktionelle Gruppen, z. B. die ε-Aminogruppe des Lysins, die Guanidinogruppe des Arginins, die Hydroxylgruppe des Threonins, Serins oder Tyrosins, oder die Mercaptogruppe des Cysteins, mit gängigen Schutzgruppen zu maskieren. Dies ist jedoch nicht in allen Fällen notwendig.

Einen wesentlichen Einfluß auf die Ausbeute an Dipeptid hat unter Umständen die Art der verwendeten Aminokomponente und ihre Konzentration im Reaktionsgemisch. Besonders leicht werden basische Aminosäuren, wie Arginin, Lysin, Ornithin und Citrullin, sowie neutrale aliphatische Aminosäuren, wie Glycin und Alanin, umgesetzt. Prolin und Hydroxyprolin als α -Iminocarbonsäuren sind der Umsetzung dagegen überhaupt nicht zugänglich. Die Konzentration der Aminokomponente hat im Gegensatz zu der der Carboxylkomponente einen wesentlichen Einfluß auf die Ausbeute an Dipeptid. Deshalb ist es vorteilhaft, die Aminokomponente in großem Überschuß einzusetzen. Der nicht verbrauchte Überschuß an Aminokomponente kann in an sich bekannter Weise leicht zurückgewonnen und erneut eingesetzt werden.

Das erfindungsgemäße Verfahren kann als Batch-Verfahren oder unter Verwendung eines geeigneten Bioreaktors kontinuierlich in einem wäßrigen oder wäßrig-organischen Puffersystem durchgeführt werden. Die erforderliche Reaktionszeit liegt im allgemeinen im Bereich zwischen 1 und 2 Stunden und hängt in erster Linie von der eingesetzten Menge an Enzym und von der Art und Konzentration der Reaktionsteilnehmer ab. Ansonsten ist die Reaktionszeit nicht kritisch, da selbst bei längerdauernder Inkubation und nach vollständigem Verbrauch der Carboxylkomponente die gebildeten Dipeptide keiner Sekundärhydrolyse durch das anwesende Enzym unterliegen.

Die Reinigung der gebildeten Dipeptide kann in an sich bekannter Weise durch Gelchromatographie, Ionenaustauscherchromatographie oder Reversed-Phase-Chromatographie erfolgen. Zur Charakterisierung, Quantifizierung und Ausbeutebestimmung können Isotachophorese und High-Performance-Liquid-Chromatographie herangezogen werden.

Die Erfindung wird durch die folgenden Beispiele näher erläutert:

### Beispiel 1:

### Synthese von Carbobenzoxy-L-alanyl-L-alanin (Z-Ala-Ala)

4 ml 1,9 M L-Alanin in 0,1 M KCl, 8 mM EDTA, 20 mM DTT wurden in ein auf 40 °C thermostatisiertes Reaktionsgefäß eingebracht, mit konzentrierter Natronlauge auf pH 9,2 eingestellt und anschließend wurden 20 mg Papain ([ EC 3.4.22.2 ] , 3,5 m Anson-E/mg) zugegeben. Die Reaktion wurde durch Zugabe von 0,2 ml 1 M Carbobenzoxy-L-alaninmethylester in Ethanol (Konzentration im Ansatz 48 mM) gestartet. Durch Zudosieren von verdünnter Natronlauge wurde der pH-Wert des Reaktionsgemisches während des Syntheseprozesses bei pH 9,2 konstant gehalten (pH-Stat-Verfahren). Nach 2 Stunden wurde die Synthese durch Zugabe von HCl (Erniedrigung des pH-Wertes auf ca. 1) gestoppt.

Zur Erfassung des Reaktionsverlaufes wurden zu verschiedenen Zeitpunkten Aliquote entnommen, mit verdünnter HCl versetzt und anschließend isotachophoretisch bzw. durch HPLC unter Verwendung einer LiChrosorb RP 18-Säule (5 µm; 250 x 4 mm) analysiert. Nach Elution mit 40 % Acetonitril/60 % 12,5 mM Phosphat-Puffer für pH 2,0 bei einer Flußrate von 1 ml/min und einer Säulentemperatur von 40 °C wurde Z-Ala-Ala durch Messung der UV-Absorption bei 210 nm detektiert.

Bezogen auf die eingesetzte Menge an Carboxylkomponente betrug die Ausbeute an Z-Ala-Ala 35 % der Theorie.

### Beispiel 2:

### Synthese von Carbobenzoxy-L-alanyl-[(S-acetamidomethyl)-L-cystein] (Z-Ala-[Acm]Cys)

Es wurde, wie in Beispiel 1 beschrieben, verfahren, jedoch enthielt das Reaktionsgemisch als Aminokomponente 4 ml 2 M S-Acetamidomethyl-L-cystein x HCl in 0,1 M KCl, 8 mM EDTA und 20 mM DTT.

Bezogen auf die eingesetzte Menge an Carboxylkomponente betrug die Ausbeute an Z-Ala-[Acm]cys 88 % der Theorie.

### Beispiel 3:

### Synthese von Carbobenzoxy-glycyl-L-alanin (Z-Gly-Ala)

Es wurde, wie in Beispiel 1 beschrieben, verfahren, jedoch enthielt das Reaktionsgemisch als Carboxylkomponente 0,2 ml 1 M Carbobenzoxy-glycinmethylester in Ethanol (Konzentration im Ansatz 48 mM).

Bezogen auf die eingesetzte Menge an Carboxylkomponente betrug die Ausbeute an Z-Gly-Ala 73 % der Theorie.

### Beispiel 4:

### Synthese von Carbobenzoxy-L-alanyl-glycin (Z-Ala-Gly)

1,8 ml 3,33 M Glycin in 0,1 M Na₂CO₃, 1 mM EDTA, 10 mM DTT (Konzentration im Ansatz 2 M) wurden in einem auf 37 °C thermostatisierten Reaktionsgefäß mit 0,6 ml Ethanol und 0,3 ml einer Papain-Lösung, entsprechend 15 mg Papain ([EC 3.4.22.2] , 3,5 m Anson-E/mg) in 0,1 M Na₂CO₃/NaHCO₃, 1 mM EDTA, 10 mM DTT, pH 9,0, gemischt und der pH-Wert auf pH 9,0 eingestellt. Die Reaktion wurde durch Zugabe von 0,3 ml 0,45 M Carbobenzoxy-L-alaninmethylester in Ethanol (Konzentration im Ansatz 45 mM) gestartet. Durch Zudosieren von 0,1 N NaOH wurde der pH-Wert des Reaktionsgemisches während des Syntheseprozesses bei pH 9,0 konstant gehalten (pH-Stat-Verfahren). Nach 2 Stunden wurde die Synthese durch Zugabe von 3 ml 10 % (w/v) Trichloracetat gestoppt.

Zur Erfassung des Reaktionsverlaufes wurden zu verschiedenen Zeitpunkten Aliquote entnommen und mit 10 % (w/v) TCA 1:2 verdünnt. Das denaturierte Protein wurde 10 Minuten bei 10.000 rpm abzentrifugiert und die Überstände 1:2 mit 25 mM Phosphatpuffer (pH 2,0)/30 % MeOH/1 % Tetrahydrofuran verdünnt und anschließend durch HPLC unter Verwendung einer Nucleosil RP 18-Säule (10 µm; 250 x 4 mm) analysiert. Nach Elution mit 29 % MeOH/1 % Tetrahydrofuran/70 % 25 mM Phosphatpuffer (pH 2,0) bei einer Flußrate von 1 ml/min und einer Säulentemperatur von 50 °C wurde Z-Ala-Gly durch Messung der UV-Absorption bei 258 nm detektiert.

Bezogen auf die eingesetzte Menge an Carboxylkomponente betrug die Ausbeute an Z-Ala-Gly 48 % der Theorie.

### Beispiel 5:

### Synthese von Carbobenzoxy-L-alanyl-L-methionin (z-Ala-Met)

Es wurde, wie in Beispiel 4 beschrieben, verfahren, jedoch enthielt das Reaktionsgemisch als Aminokomponente 1,8 ml 0,4 M L-Methionin in 0,1 M Na₂CO₃, 1 mM EDTA, 10 mM DTT (Konzentration im Ansatz 0,24 M).

Bezogen auf die eingesetzte Menge an Carboxylkomponente Betrug die Ausbeute an Z-Ala-Met 29 % der Theorie.

### Beispiel 6:

### Synthese von Carbobenzoxy-L-alanyl-L-valin (Z-Ala-Val)

Es wurde, wie in Beispiel 4 beschrieben, verfahren, jedoch enthielt das Reaktionsgemisch als Aminokomponente 1,8 ml 0,67 M L-Valin in 0,1 M Na₂CO₃, 1 mM EDTA, 10 mM DTT (Konzentration im Ansatz 0,4 M).

Bezogen auf die eingesetzte Menge an Carboxylkomponente betrug die Ausbeute an Z-Ala-Val 13 % der Theorie.

### Beispiel 7:

### Synthese von Carbobenzoxy-L-alanyl-L-arginin (Z-Ala-Arg)

Es wurde, wie in Beispiel 4 beschrieben, verfahren, jedoch enthielt das Reaktionsgemisch als Aminokomponente 1,8 ml 0,8 M L-Arginin in 0,1 M KCl, 1 mM EDTA, 10 mM DTT (Konzentration im Ansatz 0,48 M).

Bezogen auf die eingesetzte Menge an Carboxylkomponente betrug die Ausbeute an Z-Ala-Arg 72 % der Theorie.

### Beispiel 8:

Es wurde, wie in Beispiel 7 beschrieben, verfahren, jedoch enthielt das Reaktionsgemisch 0,3 ml einer Ficin-Lösung, entsprechend 3 mg Ficin ([EC 3.4.22.3] , ca. 3 U/mg, 25 °C, BAEE als Substrat) in 0,01 M Na-Acetat, 1 mM EDTA, 10 mM Cystein, pH 4,5. Die Reaktion wurde bei pH 9,0 durchgeführt

Bezogen auf die eingesetzte Menge an Carboxylkomponente betrug die Ausbeute an Z-Ala-Arg 76 % der Theorie.

### Beispiel 9:

Es wurde, wie in Beispiel 7 beschrieben, verfahren, jedoch enthielt das Reaktionsgemisch 600 mg Papain in 40 ml 0,8 M L-Arginin, 0,1 M KCl, 1 mM EDTA, 10 mM DTT, pH 9,0 und 3000 mg (12,64 mMol) Carbobenzoxy-L-alaninmethylester in 20 ml Ethanol (Konzentration im Ansatz 0,21 M). Nach 2 Stunden wurde die Synthese durch Zugabe von 60 ml 10 % (w/v) Trichloracetat gestoppt.

Bezogen auf die eingesetzte Menge an Carboxylkomponente betrug die Ausbeute an Z-Ala-Arg 71 % der Theorie.

Zur Aufarbeitung des gebildeten Carbobenzoxy-L-alanyl-L-arginins wurden 60 ml des 1:2 mit Trichloracetat verdünnten Reaktionsgemisches mittels FPLC (Pharmacia, Pep RPC HR 5/5, Wasser/Methanol, 0,5 ml/min) fraktioniert. Die Z-Ala-Arg-haltigen Fraktionen wurden vereinigt und bei 40 °C unter viermaliger Zugabe von Methanol zur Trockene eingedampft.

Ausbeute: 1360 mg (3,58 mMol) entsprechend 57 % der Theorie.

Die HPLC-Analyse zeigte, daß die weiße kristalline Substanz frei von Nebenprodukten war. Nach Hydrolyse (24 Stunden, 6N HCl) ergab die Aminosäureanalyse Ala: 1,05, Arg: 1,00.

## Patentansprüche

1. Verfahren zur enzymatischen Herstellung von Dipeptiden durch Umsetzung von N-geschützten α-Aminocarbonsäurealkylestern mit einer Aminokomponente in Gegenwart einer Cystein-Protease [EC 3.4.22] bei einem pH von mindestens 7 und bei einer Temperatur im Bereich zwischen Raumtemperatur und 60 °C, dadurch gekennzeichnet, daß man als Aminokomponente eine α-Aminocarbonsäure mit underivatisierter α-Amino- und Carboxylgruppe einsetzt.

2. Verfahren nach Anspruch 1, dadurch gekennzeichnet, daß man als Aminokomponente eine basische oder eine neutrale aliphatische α-Aminocarbonsäure einsetzt.

3. Verfahren nach Anspruch 1 oder 2, dadurch gekennzeichnet daß man die Aminokomponente in einem 1- bis 150-fachen molaren Überschuß, bezogen auf die eingesetzte Carboxylkomponente, einsetzt.

## Claims

1. A process for the enzymatic production of dipeptides by reaction of N-protected α-aminocarboxylic acid alkyl esters with an amino component in the presence of a cysteine protease [EC 3.4.22] at a pH of at least 7 and at a temperature in the range from room temperature to 60°C, characterized in that an α-aminocarboxylic acid containing an underivatized α-amino and carboxyl group is reacted as the amino component.

2. A process as claimed in claim 1, characterized in that a basic or neutral aliphatic α-aminocarboxylic acid is used as the amino component.

3. A process as claimed in claim 1 or 2, characterized in that the amino component is used in a 1- to 150-fold molar excess, based on the carboxyl component used.

## Revendications

1. Procédé de préparation enzymatique de dipeptides par réaction d'esters α-aminocarboxyliques d'alkyle N-protégés avec un composant amino en présence d'une cystéine protéase [EC.3.4.22] à un pH d'au moins 7 et à une température comprise entre la température ambiante et 60°C, caractérisé en ce qu'on utilise comme composant amino un acide α-aminocarboxylique à groupe non dérivé α-amino- et groupe carboxyle.

2. Procédé selon la revendication 1, caractérisé en ce qu'on utilise comme composant amino un acide α-amino carboxylique aliphatique basique ou neutre.

3. Procédé selon la revendication 1 ou 2, caractérisé en ce qu'on utilise le composant amino en excès molaire de 1 à 150 fois, rapporté au composant carboxyle mise en oeuvre.
